# EUROPEAN PATENT APPLICATION

(11) **EP 4 717 692 A1**
(43) Date of publication of application: **01.04.2026**
(21) Application number: 24202768.8
(22) Date of filing: 26.09.2024
(51) Int. Cl.: C07D 251/60

(54) **MELAMINE PRODUCTION PROCESS FROM PYROLYSIS OF UREA**

(71) Applicant: CASALE SA, 6900 Lugano (CH)
(72) Inventor: GAMBA, Simone, 24040 Pagazzano (BG) (IT); DI CARLO, Gabriele, 6900 Lugano (CH); SCOTTO, Andrea, 6932 Breganzona (CH)
(74) Representative: M. Zardi & Co S.A.

(57) **Abstract**

A process for the production of melamine wherein urea (1) is reacted according to a high-pressure process to form a liquid raw melamine stream (3); said raw melamine stream (3) is subjected to purification including: a first step of quenching (200) which includes mixing the raw melamine stream (3) with ammonia or with an ammonia solution (11), resulting in a quenched melamine stream (4) at a quenching temperature; a second step of polycondensates purification (300) at a purification temperature; further processing steps to produce solid melamine (10) with a desired purity; wherein the quenching temperature and the purification temperature are in a range from 135 °C to 155 °C and wherein the quenched melamine stream (4) has an ammonia content that ranges from 12% to 16% by weight.

## Description

### Field of application

The invention is in the field of industrial production of melamine from urea at high pressure without catalyst. The invention particularly relates to a process for the purification of a raw melamine stream effluent from a synthesis section, to obtain solid high-purity melamine.

### Prior art

Melamine is produced at an industrial scale starting from urea following either a non-catalytic high-pressure (HP) process or a catalytic low-pressure (LP) process. The non-catalytic high-pressure process is considered the most advantageous and is becoming predominant. In the high-pressure process, a urea melt is reacted under suitable melamine-forming conditions; the pressure is generally above 7 MPa, typically 7 to 20 MPa, and the temperature is typically around 375 °C.

The melamine synthesis section may include a single reactor or two reactors arranged in series. In the latter case, a raw melamine stream is formed in the first reactor, and the second reactor removes carbon dioxide from the melamine melt using a stripping agent, such as ammonia. This second reactor may be termed post-reactor or stripping reactor. The first reactor and the second reactor may be two separate vessels or combined in a single apparatus.

The reaction of urea to melamine produces a gaseous stream termed melamine offgas, which contains mainly ammonia and carbon dioxide, together with some melamine and other minor components. Said offgas is usually recycled as a feed material for a tied-in urea plant, after washing in a scrubber to recover the melamine contained therein. The offgas washing may be performed with urea melt or water (aqueous washing).

A combination of production of urea and melamine is attractive because urea is the reagent for the production of melamine, and the offgas released during the synthesis of melamine contains ammonia and carbon dioxide which are the starting materials for the synthesis of urea.

The raw melamine stream produced by the melamine synthesis section is usually treated in a purification section operating at lower pressure than the melamine synthesis section. Said purification section produces solid melamine with a desired purity.

The processing of the raw melamine stream includes, typically, the following steps.

The melt is firstly quenched and dissolved in a solution containing an alkaline aid such as ammonia or sodium hydroxide, forming a basic solution. Such an alkaline environment is required for carrying out the following steps.

A suitable residence time is provided to the basic solution of melamine in order to properly decompose polycondensates. During this polycondensates purification step, polycondensates are subjected to hydrolysis forming melamine and oxyaminotriazines (OAT). Quenching and polycondensates purification may be performed in two separate vessels or in a unique vessel.

The resulting solution typically passes through a filtration step and then through a crystallization step wherein solid crystals of melamine are formed. Finally, solid melamine is separated from a liquid stream, termed mother liquor, in a solid-liquid separation step. The solid melamine may be washed with water and then dried with hot air before being withdrawn from the process, whereas at least part of the mother liquor is purged and sent to a suitable treatment section; part of the mother liquor may be recycled back to quenching and/or purification.

When ammonia is used as the basic agent, the quenching step brings the temperature of the quenched melamine melt (i.e., the temperature of the basic solution of melamine) to typical values of about 170 to 180 °C, and to 120 to 140 °C when sodium hydroxide is used as basic agent. Polycondensates hydrolysis requires a suitable residence time and occurs during quenching, purification and filtration operations, stopping in the crystallization step due to the further lowering of temperature. Therefore, the total residence time dedicated to polycondensates hydrolysis is the sum of the residence times of quenching, purification and filtration. The total residence time required for a suitable hydrolysis of polycondensates depends on the content of polycondensates in the melamine melt, on the desired purity of the melamine product, and on other operating conditions such as temperature and ammonia content of the melamine solution.

Temperature plays a key role on the hydrolysis of polycondensates for kinetic reasons. Processes known in the art rely on the choice of operating parameters, such as temperature, in order to achieve the desired melamine purity, typically 99.5% or 99.8% by weight.

EP 4245755 A1 and EP 2385043 A1 disclose that the melamine melt effluent from the melamine synthesis, after quenching with ammonia as basic agent, produce a melamine solution (containing ammonia) which has a temperature between 160 °C and 180 °C, preferably between 170 °C and 172 °C.

US 3161638 discloses an impurities' purification step of a melamine melt effluent from a melamine synthesis section, after CO2 stripping from the solution produced by the quenching of said melamine melt with recycling mother liquor, the purification step being performed within a temperature range of 140 °C to 230 °C, preferably within 170 °C and 180 °C. Despite the broad temperature range of 140 °C to 230 °C, the examples given in US 3161638 specifically guide a skilled person to select a temperature within 170 °C and 180 °C.

EP 2254874 discloses a quench step to be carried out in concentrated ammonia solution between 150 °C and 190 °C, preferably between 160 °C and 180 °C.

The above-described documents of the prior art concern the treatment of a raw melamine stream coming from high-pressure melamine synthesis, in which the step of quenching said melamine stream is carried out with ammonia as basic agent. The selection of the operating parameters, particularly temperature, of the quenching and polycondensates purification, is typically aimed at obtaining a final product of solid melamine with a desired high-purity, typically 99.5% or 99.8% by weight. However, such processes could be improved if said selection of operating parameters, particularly temperatures of quenching and purification, is made by optimizing the final yield of the processes.

In addition, given the lower solubility of OATs in ammonia solutions compared to caustic soda solutions, it is crucial to properly select the temperature for melamine purification in ammonia solutions in order to minimize the formation of OATs that can co-precipitate with melamine during crystallization, resulting in a reduction of the purity of the solid melamine.

### Summary of the invention

The invention addresses the problem of how to improve the final yield of a high-purity melamine production process. More in detail, the invention addresses the problem of improving the selection of temperature values at which conducting quenching and purification from polycondensates of a liquid raw melamine stream effluent from a melamine synthesis section.

The problem is solved with a process according to the claims.

In the present invention, the process includes subjecting said raw melamine stream to a purification process aimed to produce solid melamine with a desired purity. Said purification process comprises a first step of quenching wherein the raw melamine stream is quenched by a mixing with ammonia or with an ammonia solution, resulting in a quenched melamine stream at a quenching temperature having an ammonia content that ranges from 12% to 16% by weight. The quenched melamine stream is sent to a second step of polycondensates purification wherein polycondensates contained in the quenched melamine stream are hydrolyzed by providing to said quenched melamine stream a suitable residence time at a purification temperature, resulting in a first purified melamine stream. Said purified melamine stream is then subjected to further processing steps, such as filtration, crystallization and melamine drying, in order to produce the solid melamine product with the desired purity.

According to the invention, the quenching temperature and the purification temperature range from 135 °C to 155 °C, preferably from 140 °C to 152 °C, more preferably from 145 °C to 150 °C.

The quenching and purification temperatures in a process using an ammonia solution as basic aid for quenching are conventionally selected higher than the same temperatures when a soda solution is used as basic aid. Despite that, the applicant has unexpectedly found that, even in ammonia solutions, a lower quenching and purification temperatures is surprisingly beneficial.

An advantage of melamine processes according to the invention is that, operating at lower temperatures with respect to conventional processes, they require less energy to be operated while producing solid melamine with the desired purity and with a higher yield.

### Description of the invention

The invention concerns a high-pressure process for the production of high-purity solid melamine.

Said process includes producing a raw melamine stream in a melamine synthesis process. Preferably, said raw melamine stream is substantially free from offgases derived from melamine synthesis, more preferably said raw melamine stream is substantially free from CO2. In an embodiment, offgas removal, in particular CO2 removal, is obtained by a stripping with gaseous ammonia performed in the high-pressure synthesis process.

The raw melamine stream is subjected to quenching, by mixing the raw melamine with ammonia or with an ammonia solution, resulting in a quenched melamine stream at a quenching temperature, with an increased pH required for the following purification steps.

The quenched melamine stream is sent to a step of polycondensates purification wherein polycondensates are hydrolysed at a purification temperature for a suitable residence time, resulting in a first purified melamine stream. Said first purified melamine stream is subjected to further processing steps, preferably including filtration, crystallization, solid-liquid separation and drying, so to obtain a solid melamine product with a desired purity. Said purity is preferably greater than 99.5% or 99.8% by weight.

A relevant feature of the invention it that the quenching temperature and the purification temperature are in a range from 135 °C to 155 °C, preferably from 140 °C to 152 °C, more preferably from 145 °C to 150 °C. The quenching temperature and the purification temperature may be the same or slightly different, e.g. if the quenching step and purification step are performed in separate equipment. If not the same, the quenching temperature and the purification temperature differ each other, preferably, by no more than 10 °C.

A suitable residence time is provided to the solution for the hydrolysis of polycondensates that would otherwise co-precipitate with melamine during crystallization, decreasing its purity. During quenching and purification, unreacted urea still contained in the raw melamine stream hydrolyses to give NH3 and CO2. Polycondensates decomposition occurs when the temperature is maintained substantially at the quenching or purification temperature. Therefore, the residence time during which polycondensates hydrolyse (total residence time) is the sum of the residence times for quenching, purification, and filtration steps. The residence time required for a suitable decomposition of polycondensates depends on the content of polycondensates in the raw melamine stream and on the desired melamine purity, typically 99.8% by weight for A-grade melamine or 99.5% by weight for B-grade melamine.

In a preferred embodiment of the invention, said total residence time ranges from 15 minutes to 110 minutes, preferably from 25 minutes to 90 minutes.

In a preferred embodiment of the process of the invention, the quenching and polycondensates purification are conducted in one vessel or in two separate vessels. The vessel(s) has(have) enough volume to ensure the residence time required for a suitable decomposition of polycondensates.

Preferably, the quenched melamine stream, effluent from the quenching step, is directly subjected to polycondensates purification without any other intermediate process step.

In a still preferred embodiment, the quenched melamine stream has a melamine content that ranges from 7% to 13% by weight, preferably from 8% to 11 % by weight. Moreover, the ammonia content of said stream preferably ranges from 13% to 15% by weight.

In a further embodiment, at least part of the ammonia and/or at least part of the ammonia solution used for quenching comes as a recycle from process operations downstream with respect to quenching.

In a still further preferred embodiment, the quenching and the polycondensates purification are conducted at a pressure such that ammonia is maintained in liquid phase, therefore reducing ammonia losses in vapour phase. Said pressure shall be higher the higher the quenching and purification temperatures are.

In an embodiment, the melamine crystallization is operated at a temperature between 45 °C and 50 °C, and at a pressure such that ammonia is maintained in liquid phase. Melamine crystallization and subsequent solid-liquid separation produce solid melamine and a mother liquor. Said mother liquor is at least in part purged to compensate OAT formation during the melamine synthesis and purification. The minimum purge rate of mother liquor is proportional to the OATs formed overall. Presence of ammonia in the crystallization has the aim of keeping OAT dissolved thanks to an increase of the pH, thus limiting the co-precipitation of OAT and melamine that would cause a reduction in the purity of the solid melamine product.

In a preferred embodiment, the mother liquor effluent from the solid-liquid separation step has a concentration of OATs not greater than 0.5% by weight, preferably not greater than 0.4% by weight. This limitation is meant to ensure the required purity of the solid melamine product. Given the low admissible OAT concentration (0.4% by weight or 0.5% by weight) in the mother liquor, even a small increase in OAT production in purification operations would cause a significant increase in the minimum purge flow rate and subsequent loss of melamine.

The process of the invention holds significant benefits with respect to conventional melamine processes. The choice of operating conditions of melamine purification affects both the purity of the melamine produced and the melamine yield of the process. In particular, quenching and purification temperatures play a key role as they represent the main factor influencing the kinetics of melamine and polycondensates' hydrolysis reactions. Therefore, a proper selection of said temperatures leads to significant advantages.

Hydrolysis of melamine during the purification step has two undesirable effects. Firstly, direct melamine loss by hydrolysis reaction and, secondly, indirect melamine loss due to an increase of mother liquor purge flow rate. Direct melamine loss can be limited improving the choice of operating conditions, such as quenching and purification temperatures.

The applicant has surprisingly found that by operating the quenching and purification steps between 135 °C and 155 °C, preferably from 140 °C to 152 °C, more preferably from 145 °C to 150 °C, there is a relevant reduction in direct loss of melamine by hydrolysis. In addition, at those temperatures there is also less OAT formation, resulting in less purge rate of mother liquor required to compensate said OAT formation, hence less indirect melamine loss.

The combination of lower mother liquor purge flow rate and less direct loss of melamine results in a higher global melamine yield, obtainable without reducing the purity of solid melamine produced.

Exercising quenching and purification at a lower temperature has the further advantage of a lower operating pressure required to keep the ammonia in the liquid phase, and thus lower investment cost of equipment deputed to the service.

A still further advantage of operating at lower temperature and pressure is a greater robustness and controllability of the process.

### Brief description of the figures

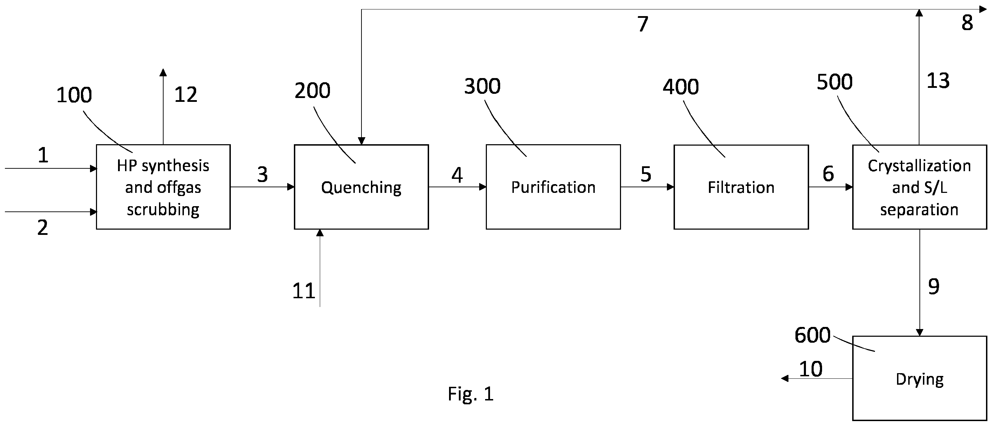

Fig. 1 shows a block scheme of a preferred embodiment of a process for producing high-purity solid melamine.

### Detailed description of the figures

Fig. 1 illustrates a schematic view of a process for producing high-purity (≥ 99.5% by weight) solid melamine 10. A raw liquid melamine stream 3 is produced from a urea melt feed 1, in presence of ammonia 2, in a synthesis section 100 operating at high pressure (7 to 20 MPa) and at a temperature around 375 °C. An offgas 12 containing CO2 and NH3, after scrubbing with urea in the same section 100, is withdrawn. The raw melamine stream 3 is substantially free of offgas and particularly of CO2 thanks to a stripping with gaseous ammonia performed in the synthesis section 100. Said raw melamine stream 3 is processed as follows to obtain the high-purity solid melamine 10.

The raw melamine stream 3 is quenched 200 with an ammonia solution 11 and with a mother liquor 7 containing ammonia, to obtain a quenched melamine stream 4 at a quenching temperature ranging from 135 °C to 155 °C, preferably from 140 °C to 152 °C, more preferably from 145 °C to 150 °C. Ammonia provides the alkalinity required by the following purification and crystallization steps. The quenched melamine stream 4 has a content of ammonia from 12% to 16% by weight and a content of melamine from 7% to 13% by weight.

The quenched melamine stream 4 is subjected to a purification step 300 wherein polycondensates are hydrolyzed by providing a suitable residence time to said stream 4, at a purification temperature from 135 °C to 155 °C, preferably from 140 °C to 152 °C, more preferably from 145 °C to 150 °C. Melamine hydrolysis and OAT formation also occur, these reactions are undesired but cannot be avoided; however, operating quenching and purification at temperatures according to the invention, melamine hydrolysis is disfavored with respect to conventional purification steps operating at higher temperatures, typically 170 °C to 180 °C.

Effluent of the purification step 300 is a purified and quenched melamine stream 5 which is then filtered 400 for the removal of dyeing substances, resulting in a filtered melamine stream 6. Filtration 400 is performed substantially at the purification temperature, therefore providing further residence time (3 to 5 minutes) for polycondensates decomposition.

The filtered melamine stream 6 is treated in a step 500 wherein temperature is decreased to 45 - 50 °C so that melamine is crystallized and separated as a solid 9 from a mother liquor 13. Said mother liquor 13 is an aqueous stream comprising water, ammonia, carbon dioxide, residual melamine and OAT, it is in part 8 processed to be purified in further process steps here not described, and in part 7 recycled to quenching 200.

The solid melamine stream 9 is finally dried 600 with hot drying air, resulting in the solid melamine 10 having the desired purity, typically 99.5% or 99.8%.

### Examples

In the following examples, the raw melamine stream produced in the synthesis section has a flow rate of 1000 kg/h, and is at a temperature of 375°C and at a pressure of 110 barg. The pressure is given in bar gauge. Said raw melamine stream is substantially free of CO2, which has been eliminated by stripping with gaseous ammonia. The composition of the melt is as follows (compositions are given in % by weight):

**Table 1: raw melamine stream to be purified.**

| | kg/h | wt. % |
|---|---|---|
| Melamine | 931.9 | 93.19 |
| OAT | 3.0 | 0.30 |
| Polycondensates | 20.0 | 2.00 |
| Urea | 10.0 | 1.00 |
| CO₂ | 0.1 | 0.01 |
| NH₃ | 35.0 | 3.50 |
| Total | 1000.0 | 100.00 |

In the following examples, the product of solid melamine has a desired purity of at least 99.8% by weight.

In the examples below, the raw melamine stream effluent from the melamine synthesis section is mixed and diluted (quenching) with 9354.4 kg/h of an ammonia solution made of water and ammonia so to obtain a quenched melamine stream having the following composition:

**Table 2: quenched melamine stream, after quenching and before purification.**

| | kg/h | wt. % |
|---|---|---|
| Melamine | 931.9 | 9.00 |
| OAT | 3.0 | 0.03 |
| Polycondensates | 20.0 | 0.19 |
| Urea | 10.0 | 0.10 |
| CO2 | 0.1 | 0.00 |
| NH3 | 1449.6 | 14.00 |
| Water | 7939.8 | 76.68 |
| Total | 10354.4 | 100.00 |

The ammonia solution is available at 50 °C and is preheated so to have the quenched melamine stream at the desired quenching temperature for purification from polycondensates.

### Example 1 - invention (140 °C)

The quenched melamine stream (Table 2) is kept at a quenching temperature of 140°C for 90 minutes for polycondensates purification, resulting in the following purified melamine stream:

**Table 3: purified melamine stream, after purification at 140 °C.**

| | kg/h | wt. % |
|---|---|---|
| Melamine | 923.2 | 8.92 |
| OAT | 33.0 | 0.32 |
| Polycondensates | 0.4 | 0.00 |
| Urea | 0.0 | 0.00 |
| CO2 | 7.5 | 0.07 |
| NH3 | 1459.1 | 14.09 |
| Water | 7931.2 | 76.60 |
| Total | 10354.4 | 100.00 |

Comparing Table 3 and Table 2, it is noticeable that polycondensates purification results in a net melamine loss of 8.7 kg/h and in an OAT formation of 30.0 kg/h. The melamine yield of the purification process, calculated as 923.2 kg/h / 931.9 kg/h x 100, is 99.07%.

The purified melamine stream of Table 3 is then subjected to crystallization at 45 °C to 50 °C, separation of solid melamine from the mother liquor, and drying of said solid melamine, to produce the following solid melamine product:

**Table 4: product of solid melamine obtained from a process with polycondensates purification operated at 140 °C.**

| | kg/h | wt. % |
|---|---|---|
| Melamine | 848.0 | 99.86 |
| OAT | 0.3 | 0.04 |
| Polycondensates | 0.4 | 0.05 |
| Urea | 0.0 | 0.00 |
| CO2 | 0.0 | 0.00 |
| NH3 | 0.0 | 0.00 |
| Water | 0.4 | 0.05 |
| Total | 849.1 | 100.00 |

The product of solid melamine has a purity not less than 99.8% by weight, and the overall melamine process yield, calculated as 849.1 kg/h / 1000 kg/h x 100, is 84.9%. The mother liquor effluent from solid-liquid separation comprises dissolved melamine at 0.8% by weight and OAT at 0.35% by weight. The melamine lost in said mother liquor, and therefore not recovered from crystallization, is 75.3 kg/h.

In order to lead the quenching temperature to 140 °C, a power of 877.3 kW is required to preheat the ammonia solution from 50 °C to 128.1 °C.

The minimum working pressure, at 140 °C, to keep the melamine streams in liquid phase is 9.7 barg.

### Example 2 - conventional process (170 °C)

The quenched melamine stream (Table 2) is kept at a quenching temperature of 170 °C for 45 minutes for polycondensates purification, resulting in the following purified melamine stream:

**Table 5: purified melamine stream, after purification at 170 °C.**

| | kg/h | wt. % |
|---|---|---|
| Melamine | 908.8 | 8.78 |
| OAT | 47.8 | 0.46 |
| Polycondensates | 0.0 | 0.00 |
| Urea | 0.0 | 0.00 |
| CO2 | 7.8 | 0.08 |
| NH3 | 1463.7 | 14.14 |
| Water | 7926.3 | 76.54 |
| Total | 10354.4 | 100.00 |

Comparing Table 5 and Table 2, it is noticeable that polycondensates purification results in a net melamine loss of 23.1 kg/h and in an OAT formation of 44.8 kg/h. The melamine yield of the purification process, calculated as 908.8 kg/h / 931.9 kg/h x 100, is 97.52%.

The purified melamine stream of Table 5 is then subjected to crystallization at 45 °C to 50 °C, separation of solid melamine from the mother liquor, and drying of said solid melamine, to produce the following solid melamine product:

**Table 6: product of solid melamine obtained from a process with polycondensates purification operated at 170 °C.**

| | kg/h | wt. % |
|---|---|---|
| Melamine | 833.3 | 99.89 |
| OAT | 0.5 | 0.06 |
| Polycondensates | 0.006 | 0.00 |
| Urea | 0.0 | 0.00 |
| CO2 | 0.0 | 0.00 |
| NH3 | 0.0 | 0.00 |
| Water | 0.4 | 0.05 |
| Total | 834.2 | 100.00 |

The product of solid melamine has a purity nor less than 99.8% by weight, and the overall melamine process yield, calculated as 834.2 kg/h / 1000 kg/h x 100, is 83.4%. The mother liquor effluent from solid-liquid separation comprises dissolved melamine at 0.8% by weight and OAT at 0.5% by weight. The melamine lost in said mother liquor, and therefore not recovered from crystallization, is 75.4 kg/h.

In order to lead the quenching temperature to 170 °C, a power of 1285.4 kW is required to preheat the ammonia solution from 50 °C to 160.3°C.

The minimum working pressure, at 170 °C, to keep the melamine streams in liquid phase is 18.5 barg.

### Example 3 - invention (150 °C; case 1)

The quenched melamine stream (Table 2) is kept at a quenching temperature of 150 °C for 55 minutes for polycondensates purification, resulting in the following purified melamine stream:

**Table 7: purified melamine stream, after purification at 150 °C.**

| | kg/h | wt. % |
|---|---|---|
| Melamine | 924.6 | 8.93 |
| OAT | 31.6 | 0.31 |
| Polycondensates | 0.4 | 0.00 |
| Urea | 0.0 | 0.00 |
| CO2 | 7.5 | 0.07 |
| NH3 | 1459.0 | 14.09 |
| Water | 7931.3 | 76.60 |
| Total | 10354.4 | 100.00 |

Comparing Table 7 and Table 2, it is noticeable that polycondensates purification results in a net melamine loss of 7.3 kg/h and in an OAT formation of 28.6 kg/h. The melamine yield of the purification process, calculated as 924.6 kg/h / 931.9 kg/h x 100, is 99.22%.

The purified melamine stream of Table 7 is then subjected crystallization at 45 °C to 50 °C, separation of solid melamine from the mother liquor, and drying of said solid melamine, to produce the following solid melamine product:

**Table 8: product of solid melamine obtained from a process with polycondensates purification operated at 150 °C.**

| | kg/h | wt. % |
|---|---|---|
| Melamine | 849.3 | 99.86 |
| OAT | 0.3 | 0.04 |
| Polycondensates | 0.4 | 0.05 |
| Urea | 0.0 | 0.00 |
| CO2 | 0.0 | 0.00 |
| NH3 | 0.0 | 0.00 |
| Water | 0.4 | 0.05 |
| Total | 850.4 | 100.00 |

The product of solid melamine has a purity not less than 99.8% by weight, and the overall melamine process yield, calculated as 850.4 kg/h / 1000 kg/h x 100, is 85.0%. The mother liquor effluent from solid-liquid separation comprises dissolved melamine at 0.8% by weight and OAT at 0.33% by weight. The melamine lost in said mother liquor, and therefore not recovered from crystallization, is 75.3 kg/h.

In order to lead the quenching temperature to 150 °C, a power of 1010.4 kW is required to preheat the ammonia solution from 50 °C to 138.9 °C.

The minimum working pressure, at 150 °C, to keep the melamine streams in liquid phase is 12.2 barg.

### Example 4 - invention (150 °C; case 2)

The quenched melamine stream (Table 2) is kept at a quenching temperature of 150 °C for 40 minutes for polycondensates purification, resulting in the following purified melamine stream:

**Table 9: purified melamine stream, after purification at 150 °C.**

| | kg/h | wt. % |
|---|---|---|
| Melamine | 928.4 | 8.97 |
| OAT | 27.1 | 0.26 |
| Polycondensates | 1.0 | 0.01 |
| Urea | 0.0 | 0.00 |
| CO2 | 7.4 | 0.07 |
| NH3 | 1458.0 | 14.08 |
| Water | 7932.5 | 76.61 |
| Total | 10354.4 | 100.00 |

Comparing Table 9 and Table 2, it is noticeable that polycondensates purification results in a net melamine loss of 3.5 kg/h and in an OAT formation of 24.1 kg/h. The melamine yield of the purification process, calculated as 928.4 kg/h / 931.9 kg/h x 100, is 99.62%.

The purified melamine stream of Table 9 is then subjected to crystallization at 45 °C to 50 °C, separation of solid melamine from the mother liquor, and drying of said solid melamine, to produce the following solid melamine product:

**Table 10: product of solid melamine obtained from a process with polycondensates purification operated at 150 °C.**

| | kg/h | wt. % |
|---|---|---|
| Melamine | 853.2 | 99.80 |
| OAT | 0.3 | 0.03 |
| Polycondensates | 1.0 | 0.12 |
| Urea | 0.0 | 0.00 |
| CO2 | 0.0 | 0.00 |
| NH3 | 0.0 | 0.00 |
| Water | 0.4 | 0.05 |
| Total | 854.9 | 100.00 |

The product of solid melamine has a purity not less than 99.8% by weight, and the overall melamine process yield, calculated as 854.9 kg/h / 1000 kg/h x 100, is 85.5%. The mother liquor effluent from solid-liquid separation comprises dissolved melamine at 0.8% by weight and OAT at 0.29% by weight. The melamine lost in said mother liquor, and therefore not recovered from crystallization, is 75.2 kg/h.

Comparing the two cases at the same temperature (examples 3 and 4 at 150°C), it is possible to notice that melamine with the desired purity may be obtained even for major variations of residence time and with small variations in OAT formation. This implies a robust process that is easy to control and optimize at the industrial level even when load changes are made in the plant itself. A non-negligible variation in residence time causes neither a significant increase in OAT production, which could generate problems during crystallization, nor the immediate production of out-of-specification melamine by increase beyond the limit of residual polycondensates. Therefore, a process for the purification of melamine in ammonia solutions according to the invention represents a more robust process that more easily preserves the consistency of the quality of the final product.

### Example 5 - conventional process (172 °C)

The quenched melamine stream (Table 2) is kept at a quenching temperature of 172 °C for 30 minutes for polycondensates purification, resulting in the following purified melamine stream:

**Table 11: purified melamine stream, after purification at 172 °C.**

| | kg/h | wt. % |
|---|---|---|
| Melamine | 917.9 | 8.86 |
| OAT | 38.7 | 0.37 |
| Polycondensates | 0.1 | 0.00 |
| Urea | 0.0 | 0.00 |
| CO2 | 7.6 | 0.07 |
| NH3 | 1461.2 | 14.11 |
| Water | 7928.9 | 76.59 |
| Total | 10354.4 | 100.00 |

Comparing Table 11 and Table 2, it is noticeable that polycondensates purification results in a net melamine loss of 14.0 kg/h and in an OAT formation of 35.7 kg/h. The melamine yield of the purification process, calculated as 917.9 kg/h / 931.9 kg/h x 100, is 98.5%.

The purified melamine stream of Table 11 is then subjected to crystallization at 45 °C to 50 °C, separation of solid melamine from the mother liquor, and drying of said solid melamine, to produce the following solid melamine product:

**Table 12: product of solid melamine obtained from a process with polycondensates purification operated at 172 °C.**

| | kg/h | wt. % |
|---|---|---|
| Melamine | 842.5 | 99.90 |
| OAT | 0.4 | 0.05 |
| Polycondensates | 0.055 | 0.00 |
| Urea | 0.0 | 0.00 |
| CO2 | 0.0 | 0.00 |
| NH3 | 0.0 | 0.00 |
| Water | 0.4 | 0.05 |
| Total | 843.4 | 100.00 |

The product of solid melamine has a purity nor less than 99.8% by weight, and the overall melamine process yield, calculated as 843.4 kg/h / 1000 kg/h x 100, is 84.3%. The mother liquor effluent from solid-liquid separation comprises dissolved melamine at 0.8% by weight and OAT at 0.41% by weight. The melamine lost in said mother liquor, and therefore not recovered from crystallization, is 75.4 kg/h.

In order to lead the quenching temperature to 172 °C, a power of 1313.5 kW is required to preheat the ammonia solution from 50 °C to 162.5 °C.

The minimum working pressure, at 172 °C, to keep the melamine streams in liquid phase is 19.3 barg.

Comparing the two examples at 170 °C and 172 °C it is possible to notice that a 15 min increase in residence time, despite accompanied by a 2 °C decrease in temperature, causes an increase in OAT generation that is double that of the 150°C cases (comparing the difference 44.8 kg/h - 35.7 kg/h = 9.1 kg/h with the difference 28.6 kg/h - 24.1 kg/h = 4.5 kg/h). This implies a process at 170 - 172 °C that is much more sensitive to changes in residence time than cases at lower temperatures and thus a process that is more difficult to control under the aspect of OAT generation (less robust process).

### Considerations about examples 1 - 5

A process according to an embodiment of the invention (example No. 1 at 140 °C), compared to a conventional process known in the art (example No. 2 at 170 °C), ensures that high-purity melamine (≥ 99.8% by weight) is obtained with a higher final yield, less OAT generation, and less melamine loss during purification. In addition, the process of the invention requires lower pressures and operating temperatures, and thus less expensive equipment.

Comparing the conventional process of example 2 with the process according to an embodiment of the invention of example 4 it is noticeable that the process of the invention, even with essentially a lower residence time, can still allow to obtain high-purity melamine with better yield and less OAT generation, thanks to its lower quenching temperature (150 °C).

Furthermore, starting from a solution containing the same concentration of melamine, OAT and polycondensates, conventional processes produce mother liquors (effluent from solid-liquid separation after crystallization) that are richer in OAT. Therefore, in such processes any variation regarding, for example, the quality of the melamine melt, the concentration of ammonia in the solution to be purified and thus in the crystallization mother liquor, the temperature or residence time for purification, is more likely to lead to the production of solid melamine with a lower purity (even off-specification), due to co-precipitation of OATs present in excess of the solubility limit at the crystallization temperature (45 °C to 50 °C).

The above-examples show also how conventional processes require more energy expenditure, with respect to a process of the invention, to maintain its higher quenching temperature.

### Example 6 - process at low temperature (130 °C)

The melamine melt of Table 1 was diluted with 11425.3 kg/h of an ammonia solution (consisting only of water and ammonia) in order to obtain a melamine solution diluted enough to take into account for the lower solubility of melamine at lower temperatures:

**Table 113: quenched melamine stream, after quenching at 130 °C and before purification.**

| | kg/h | wt. % |
|---|---|---|
| Melamine | 931.9 | 7.50 |
| OAT | 3.0 | 0.02 |
| Polycondensates | 20.0 | 0.16 |
| Urea | 10.0 | 0.08 |
| CO2 | 0.1 | 0.00 |
| NH3 | 1739.5 | 14.00 |
| Water | 9720.9 | 78.24 |
| Total | 12425.3 | 100.00 |

The quenched melamine stream (Table 13) is kept at a quenching temperature of 130 °C for 155 minutes for polycondensates purification, resulting in the following purified melamine stream:

**Table 14: purified melamine stream, after purification at 130 °C.**

| | kg/h | wt. % |
|---|---|---|
| Melamine | 921.3 | 7.42 |
| OAT | 35.0 | 0.28 |
| Polycondensates | 0.4 | 0.00 |
| Urea | 0.0 | 0.00 |
| CO2 | 7.5 | 0.06 |
| NH3 | 1749.4 | 14.08 |
| Water | 9711.7 | 78.16 |
| Total | 12425.3 | 100.00 |

Comparing Table 14 and Table 13, it is noticeable that polycondensates purification results in a net melamine loss of 10.6 kg/h and in an OAT formation of 32.0 kg/h. The melamine yield of the purification process, calculated as 921.3 kg/h / 931.9 kg/h x 100, is 98.86%.

The purified melamine stream of Table 14 is then subjected to crystallization at 45 °C to 50 °C, separation of solid melamine from the mother liquor, and drying of said solid melamine, to produce the following solid melamine product:

**Table 15: product of solid melamine obtained from a process with polycondensates purification operated at 130 °C.**

| | kg/h | wt. % |
|---|---|---|
| Melamine | 829.2 | 99.87 |
| OAT | 0.3 | 0.03 |
| Polycondensates | 0.4 | 0.05 |
| Urea | 0.0 | 0.00 |
| CO2 | 0.0 | 0.00 |
| NH3 | 0.0 | 0.00 |
| Water | 0.4 | 0.05 |
| Total | 830.3 | 100.00 |

The product of solid melamine has a purity nor less than 99.8% by weight, and the overall melamine process yield, calculated as 830.3 kg/h / 1000 kg/h x 100, is 83.0%. The mother liquor effluent from solid-liquid separation comprises dissolved melamine at 0.8% by weight and OAT at 0.30% by weight. The melamine lost in said mother liquor, and therefore not recovered from crystallization, is 92.0 kg/h.

In order to lead the quenching temperature to 130 °C, a power of 945.8 kW is required to preheat the ammonia solution from 50 °C to 119.6 °C.

Quenching at 130 °C, the total purging of the mother liquor, due to the higher initial dilution required to keep the melamine in solution, results in a greater melamine loss. Despite the higher initial dilution and total purging, the mother liquor still has a higher OAT concentration than in the 150 °C case 2 (Example 4) due to the worse performance of the purification process in terms of melamine loss and OAT generation.

Melamine losses in the mother liquor can be decreased by using part of the mother liquor for quenching, instead of ammonia solution alone. However, being the purification process less efficient at lower temperatures, even if a mother liquor recycle is applied, the higher OAT generation results in a higher minimum purge rate required. Thus, the lower-temperature processes would still have a lower overall solid melamine yield than the cases covered by the invention.

Furthermore, at lower quenching temperatures, the residence time required for purification and the flow rate of the solution to be purified increase significantly due to kinetic reasons and a lower solubility of melamine. The above two facts require the use of larger volume equipment, resulting in higher investment costs. This increase in equipment volume has a greater impact on overall costs than the decreased operating temperature and pressure.

The higher flow rate of ammonia solution required for melamine melt dilution results in higher process energy expenditure, despite the lower operating temperatures.

The lower quenching temperature results in a higher energy expenditure (pumps) for circulating a higher flow rate of melamine solution.

The significant increase of the required residence time results in greater melamine loss and greater OAT generation, despite the lower temperature and lower initial melamine concentration.

## Claims

1. A process for the production of melamine including:
urea (1) is reacted according to a non-catalytic high-pressure melamine synthesis process (100), producing a gaseous stream (12) and a liquid raw melamine stream (3);
said raw melamine stream (3) is subjected to a purification process including: a first step of quenching (200) which includes mixing the raw melamine stream (3) with ammonia or with an ammonia solution (11), resulting in a quenched melamine stream (4) at a quenching temperature; a second step of polycondensates purification (300) wherein polycondensates contained in the quenched melamine stream (4) are hydrolysed at a purification temperature for a suitable residence time, resulting in a first purified melamine stream (5); said first purified melamine stream being subjected to further processing steps in order to produce solid melamine (10) with a desired purity;
wherein the quenching temperature and the purification temperature are in a range from 135 °C to 155 °C and wherein the quenched melamine stream (4) has an ammonia content that ranges from 12% to 16% by weight.

2. A process according to claim 1 wherein the quenching temperature and the purification temperature are in a range from 140 °C to 152 °C, preferably from 145 °C to 150 °C.

3. A process according to claim 1 or 2 wherein the quenching temperature and the purification temperature differ each other by no more than 10 °C.

4. A process according to any of the previous claims wherein the desired purity of the produced solid melamine (10) is greater than 99.5% by weight, preferably greater than 99.8% by weight.

5. A process according to any of the previous claims wherein the quenching (200) and polycondensates purification (300) are conducted in one vessel or in two separate vessels.

6. A process according to any of the previous claims wherein the quenched melamine stream (4), effluent from the quenching step (200), is directly subjected to polycondensates purification (300) without any other intermediate process step.

7. A process according to any of the previous claims wherein the quenched melamine stream (4) has a melamine content that ranges from 7% to 13% by weight, preferably from 8% to 11% by weight.

8. A process according to any of the previous claims wherein the quenched melamine stream (4) has an ammonia content that ranges from 13% to 15% by weight.

9. A process according to any of the previous claims wherein the raw melamine stream (3) is substantially free from offgas coming from melamine synthesis.

10. A process according to any of the previous claims wherein the raw melamine stream (3) is substantially free from CO2.

11. A process according to any of the previous claims wherein offgas removal from the raw melamine stream, in particular CO2 removal, is obtained by a stripping operation with gaseous ammonia, said stripping operation being performed in the high-pressure melamine synthesis process (100).

12. A process according to any of the previous claims wherein at least part of the ammonia and/or at least part of the ammonia solution (11) used for quenching (200) comes as a recycle from process operations carried out downstream with respect to quenching.

13. A process according to any of the previous claims wherein the quenching (200) and the polycondensates purification (300) are conducted at a pressure such that ammonia is maintained in liquid phase.

14. A process according to any of the previous claims wherein the further processing steps of the first purified melamine stream (5) include a filtration (400), melamine crystallization, subsequent solid-liquid separation (500), to produce a mother liquor and a solid wet melamine, said solid wet melamine being subjected to melamine drying (600) resulting in the solid melamine (10) effluent from the process.

15. A process according to claim 14 wherein the quenching (200), polycondensates purification (300) and filtration (400) provide a residence time for polycondensates decomposition between 15 minutes and 110 minutes, preferably between 25 minutes and 90 minutes.

16. A process according to claim 14 or 15 wherein the melamine crystallization step (500) is conducted at a temperature between 45 °C and 50 °C, and at a pressure such that ammonia is maintained in liquid phase.

17. A process according to any of claims 14 to 16 wherein the mother liquor is at least in part recycled in the step of quenching (200) of the raw melamine stream (3) and the quenching step includes mixing the raw melamine with the recycled mother liquor (7).

18. A process according to any of claims 14 to 17 wherein the mother liquor (13) effluent from the solid-liquid separation step (500) has a concentration of OATs not greater than 0.5% by weight, preferably not greater than 0.4% by weight.
